# EUROPEAN PATENT SPECIFICATION

(11) **EP 1 601 361 B1**
(45) Date of publication and mention of the grant of the patent: **14.05.2008**
(21) Application number: 04716605.3
(22) Date of filing: 03.03.2004
(51) Int. Cl.: A61K 31/505, A61P 13/12

(54) **PYRIDYLSULFONAMIDO PYRIMIDINES FOR TREATING DIABETIC NEPHROPATHY**
PYRIDLSULFONAMIDOPYRIMIDINE ZUR BEHANDLUNG VON DIABETISCHER NEPHROPATHIE
PYRIMIDINES PYRIDYLSULFONAMIDIQUES POUR LE TRAITEMENT DE LA NEPHROPATHIE DIABETIQUE

(30) Priority: 06.03.2003 EP 03100549
(43) Date of publication of application: 07.12.2005
(73) Proprietor: Speedel Pharma AG, 4051 Basel (CH)
(72) Inventor: MANN, Jessica, Basel, 4051 (CH)
(74) Representative: Maué, Paul Georg
(86) International application number: PCT/EP2004/050242
(87) International publication number: WO 2004/078104

(56) References cited:
- WO-A-01/81335
- US-B1- 6 417 360
- BENIGNI A; COLOSIO V; BRENA C; BRUZZI I; BERTANI T; REMUZZI G: "Unselective Inhibition of Endothelin Receptors Reduces Renal Dysfunction in Experimental Diabetes" DIABETES, vol. 47, no. 3, 1 March 1998 (1998-03-01), pages 450-456, XP001180574
- SUGIMOTO KOH-ICHI; TSURUOKA SHUICHI; FUJIMURA AKIO: "Renal protective effect of YM598, a selective endothelin ET(A) receptor antagonist, against diabetic nephropathy in OLETF rats." EUROPEAN JOURNAL OF PHARMACOLOGY, vol. 450, no. 2, 23 August 2002 (2002-08-23), pages 183-189, XP002288658 NETHERLANDS
- KELLY ET AL: "Effects of endothelin or angiotensin II receptor blockade on diabetes in the transgenic (mRen-2)27 rat" KIDNEY INTERNATIONAL, vol. 57, May 2000 (2000-05), pages 1882-1894,
- CAO ET AL: "Blockade of the renin-angiotensin and endothelin systems on progressive renal injury" HYPERTENSION, vol. 36, no. 4, October 2000 (2000-10), pages 561-568,
- YANG SHENGZU; LITCHFIELD JOHN E; BAYNES JOHN W: "AGE-breakers cleave model compounds, but do not break Maillard crosslinks in skin and tail collagen from diabetic rats." ARCHIVES OF BIOCHEMISTRY AND BIOPHYSICS, vol. 412, no. 1, April 2003 (2003-04), pages 42-46,

## Description

The present invention relates to a new medicament for the treatment of diabetic nephropathy comprising the use of specific pyridylsulfonamido pyrimidines.

Diabetic nephropathy is the principle cause of and stage renal disease in the western world. It is a major cause of morbidity and mortality in Type-I Diabetes, but is an increasing problem in Type-II Diabetes and because the incidence of this is five times that of Type-I Diabetes, it contributes at least 50% of diabetics with end stage renal disease.

The initial stage of subtle morphologic changes in the renal glomeruli is followed by microalbuminuria. This is associated with a modestly rising blood pressure and an increased Incidence of cardiovascular disease. There follows a continued increase in urinary protein excretion and defining glomerular filtration rate. Diabetic nephropathy has many possible underlying pathophysiological causes including metabolic, glycosylation of proteins, haemodynamics, altered flow/pressure in glomeruli, the development of hypertension and cytokine production; all of these are associated with the development of extra cellular matrix and increased vascular permeability leading to glomerular damage and proteinuria.

A number of publications provide evidence for the predictive value of proteinuria as the single most important factor to predict progression of renal dysfunction and in particular diabetic nephropathy; W.F. Keane et al., Proteinuria, Albuminuria, Risk, Assessment, Detection, Elimination (PARADE): A Position paper of the National Kidney Foundation, American J. of Kidney Diseases, Vol. 33, May 1999, pp1004-1010*.* In addition persistent proteinuria or albuminurla has been shown to indicate an increased risk for acute coronary events and for stroke. Studies investigating losartan and irbesartan (belonging to the class of angiotensin receptor blockers) show a decrease in proteinuria correlating with a reduction in onset of end-stage renal disease but no change in mortality rates; M. Brenner et al. Effects of losartan on renal and cardiovascular outcomes in patients with type 2 diabetes and nephropathy. N Engl J Med 2001;345:861- 869 and E.J. Lewis et al. Renoprotective effect of the angiotensin-receptor irbesartan in patients with nephropathy due to type 2 diabetes. N Engl J Med 2001;345:851- 860*..* Thus, there is a recognised still not fully met medical need to control or reduce proteinuria, and to reduce both mortality rates and frequency of end stage renal disease, in particular in addition to an existing treatment.

The sulfonamides of the present invention are known as inhibitors of endothelin receptors from US 6,417,360 and WO 00/52007.

WO01/81335 discloses tetra-substituted pyrimidine-sulfonamides having endothelin-antagonist activity whereby as precursors chloro compounds according to the present invention may be used.

A. Benigni, V. Colosio, C. Brenca, I. Bruzzi, T. Bertani and G. Remuzzi in "Unselective Inhibition of Endothelin Receptors Reduces Renal Dysfunction in Experimental Diabetes", Diabetes, vol. 47, March 1998, pages 450-456, report studies of the unselective ET_{A}/ET_{B} receptor antagonist PD 142,893 in a diabetic rat model and conclude that the results support that ET-1 is at least one contributory mediator of kidney damage in diabetes.

K.-I. Sugimoto, S. Tsuruoka and A. Fujimura in "Renal protective effect of YM598, a selective endothelin ETA receptor antagonist, against diabetic nephropathy in OLETF rats", Europ. J. of Pharmacology, vol. 450, August 2002, pages 183-189, report studies of the selective ET_{A} receptor antagonist YM598 in a type II diabetes rat model and conclude that an endothelin ET_{A} receptor antagonist may be useful for the treatment of diabetic nephropathy.

The present invention relates to compounds of formula (I) 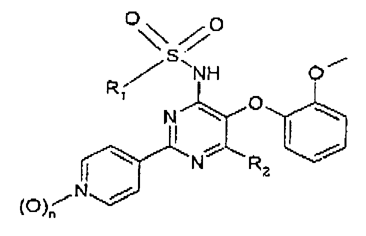 wherein
R; is pyridyl or thiazolyl, any of which may optionally be substituted with C₁₋₈alkyl or C₂₋₈alkenyl; and
a) R₂ is methoxy and n is zero or one; or
b) R₂ is chlorine and n is zero
and pharmaceutically acceptable salts thereof,
which surprisingly show a significant proteinuria lowering effect, in particular when administered to patients with Type-II diabetes,

The present invention relates to the use of a compound of formula (I) for the manufacture of a medicament for lowering or controlling proteinuria, in particular for the treatment of diabetic nephropathy, be it in patients with type I or type II diabetes,
preferably, human beings or a mammalian animals.

The term "treatment" as used throughout the description of the instant invention is meant to Include also "prevention" and delay of progression". In particular, the term "treatment" comprises the reduction in mortality rates.

The sulfonamides of the present invention are known as inhibitors of endothelin receptor and a method of preparation is disclosed in WO 00/52007.

More particularly, the present invention relates to the following compounds of formula (I): R₁ is preferably, optionally substituted with C₁₋₈alkyl or G₂₋₈alkenyl, 2-pyridyl or 2-thiazolyl and most preferably, optionally substituted with C₁₋₈alkyl or C₂₋₈alkenyl. 2-pyridyl. C₁₋₈alkyl or C₂₋₈alkenyl are branched or Straight chain radicals, for example methyl, ethyl, n-propyl, Isopropyl, n-butyl, isobutyl, t-butyl, vinyl. 1-propenyl, allyl, isopropenyl, 1-butenyl, 2-butenyl, 3-butenyl and the like. Preferred are said residues which have up to (and including) four carbon atoms. Most preferred is methyl.

Particularly preferred are compounds of formula (I) wherein
R₁ is 2-pyridyl optionally substituted with C₁₋₄alkyl; and
R₂ is methoxy and n is zero
and pharmaceutically acceptable salts thereof.

Most preferred is 5-methyl-pyridine-2-sulfonic acid [6-methoxy-5-(2-methoxy-phenoxy)-2-pyridin-4-yl-pyrimidin-4-yl]-amide.

The term "pharmaceutically acceptable salts" comprises salts of the compounds of formula (I) with inorganic or organic acids such as hydrochloric acid, hydrobromic acid, nitric acid, sulphuric acid, phosphoric acid, citric acid, formic acid, maleic acid, acetic acid, succinic acid, tartaric acid, methanesulphonic acid, p-toluenesulphonic acid and the like, which are nontoxic to living organisms. It also includes salts with inorganic or organic bases such as alkali salts like sodium and potassium salts, alkaline earth metal salts like calcium and magnesium salts, N-methyl-D-glutamine salts and salts with amino acids like arginine, lysine and the like.

It will be appreciated that the compounds of formula (I) in this invention may be derivatised at functional groups to provide prodrug derivatives that are capable of conversion back to the parent compounds in vivo. Additionally, any physiologically acceptable equivalents of the compounds of general formula (I), which are capable of producing the parent compounds of general formula (I) in vivo, are within the scope of this invention.

As mentioned earlier, the use of a compound of formula (I) for the manufacture of a medicament for the treatment of diabetic nephropathy is an object of the instant invention, which manufacture comprises bringing one or more compounds of formula (I) and, if desired, one or more other therapeutically valuable substances into a pharmaceutical administration form.

The pharmaceutical compositions may be administered orally, for example in the form of tablets, coated tablets, dragees, hard or soft gelatine capsules, solutions, emulsions or suspensions. Administration can also be Carried out rectally, for example using suppositories; locally or percutaneously, for example using ointments, Creams, gels or solutions; or parenterally, e.g. intravenously, intramuscularly, subcutaneously, intrathecally or transdermally, using for example injectable solutions- Furthermore, administration can be carried out sublingually or as opthalmological preparations or as an aerosol, for example in the form of a spray.

For the preparation of tablets, coated tablets, dragees or hard gelatine capsules the compounds of the present invention may be mixed with pharmaceutically inert, inorganic or organic excipients. Examples of suitable excipients for tablets, dragees or hard gelatine capsules include lactose, maize starch or derivatives thereof, talc or stearic acid or salts thereof.

Suitable excipients for use with soft gelatine capsules may include for example vegetable oils, waxes, fats, semi-solid or liquid polyols etc..

For the preparation of solutions and syrups, excipients which may be used include for example water, polyols, saccharose, invert sugar and glucose.

For injectable solutions, excipients which may be used include for example water, alcohols, polyols, glycerine, and vegetable oils.

For suppositories, and local Or percutaneous application, excipients which may be used include for example natural or hardened oils, waxes, fats and semi-solid or liquid polyols.

### The following examples illustrate possible administration forms:

Tablets containing the following ingredients can be produced in a conventional manner;

| Ingredients | mg per tablet |
|---|---|
| Compound of formula (I) | 10.0-100.0 |
| Lactose | 125.0 |
| Corn starch | 75.0 |
| Talc | 4.0 |
| Magnesium stearate | 1.0 |

### Capsules containing the following ingredients can be produced in a conventional manner

| Ingredients | mg per capsule |
|---|---|
| Compound of formula (I) | 25.0 |
| Lactose | 150.0 |
| Corn starch | 20.0 |
| Talc | 5.0 |

### Injection solutions can have the following compositions:

| | |
|---|---|
| Compound of formula (I) | 1,0 mg |
| Sodium Chloride | 8,5 mg |
| Tris (hydroxymethyl) aminomethane | 0.5 mg |
| 0.1 N HCl | ad PH 8 |
| Water for injection | ad 1.0 ml |

The pharmaceutical compositions may also contain preserving agents, Solubilising agents, stabilising agents, wetting agents, emulsifiers, sweeteners, colourants, odorants, salts for the variation of osmotic pressure, buffers, coating agents or antioxidants. As mentioned earlier, they may also contain other therapeutically valuable agents,

It is a prerequisite that all adjuvants used in the manufacture of the preparations are generally recognized as safe.

Preferred forms of use are intravenous, intramuscular or oral administration, most preferred is oral administration. The dosages in which the compounds of formula (I) are administered in effective amounts depend on the nature of the specific active ingredient, the age and the requirements of the patient and the mode of application. In general, dosages of about 0.01-10 mg/kg body weight per day come into consideration.

The compounds of formula (I) may also be administered in combination with antihypertensive drugs, antiarrhythmics, anti anginal, protein kinase inhibitors and/or modulators, urotensin II receptor antagonists, drugs which act on proteins such as fibrinogen and matrix metalloproteinases, antithrombotics, lipid lowering agents, antioxidants and, preferred, any drugs which act on the renin-angiotensin system such as angiotensin-converting enzyme inhibitors (ACEIs; such as captopril and benazepril), renin inhibitors ( such as aliskiren), aldose reductase inhibitors (such as AS-3201), proteinkinase C beta- inhibitors (such as ruboxistaurin), AGE crosslink breakers/inhibitors (such as pyridoxamine or ALT-711), heparin type molecules (such as sulodexide), aldosterone receptor antagonists (such as eplerenone or spironolactone) and, particularly preferred, angiotensin receptor blockers (ARBs). Examples of ARBs are, among others, eprosartan, oimesartan, tasosartan, telmisartan, irbesartan, valsartan, candesartan and losartan. Said ARBs may be used at high doses in which case a high dose, by way of example, corresponds to 300 mg od irbesartan, 160 mg od valsartan, 32 mg od candesartan or 50 mg bid losartan.

For above compounds which may be administered in combination with a compound of formula (I), preference is given to commercially available compounds or those compounds which have been approved by a health authority. Consequently, a further object of the instant invention is

a pharmaceutical composition comprising a compound of formula (I), an ARB. an ACEI, a renin inhibitor, an aldose reductase inhibitor, a proteinkinase C beta- inhibitor, an AGE crosslink breaker/inhibitor, a heparin type molecule or an aldosterone receptor antagonist and an excipient.

A further object of the instant invention is a kit for the treatment of diabetic nephropathy comprising
a) an amount of the compound of formula (I) in a first unit dosage form:
b) an amount of at least one therapeutic agent selected from the group consisting of ARBs, ACEIs, renin inhibitors, aldose reductase inhibitors, proteinkinase C beta- inhibitors. AGE crosslink breakers/inhibitors, heparin type molecule and aldosterone receptor antagonists in a second etc. unit dosage form, and
c) a container containing said first, second etc. unit dosage forms.

In a variation thereof, the instant invention likewise relates to a "kit-of-parts", in the sense that the components to be combined according to the instant invention can be dosed independently or by use of different fixed combinations with distinguished amounts of the components, i.e. simultaneously or at different time points.

The effectiveness of the compounds of formula (I) on reducing or controlling proteinuria and in particular on diabetic nephropathy can be demonstrated using animal models, known to the person skilled in the art, or the procedure described hereafter in the Example.

Thus, for example, the short term and long term effects of the compounds of formula (I) on the development of glomerular lesions can be determined after administration of the test compound to hyperglycaemic diabetic rats, the method used is analogous to the test method described in J. Am. Nephrol. 1993. 4:40-49. A therapeutic effect is present, for example, when, in such diabetic rats, the increase in the glomerular filtration rate is prevented and proteinuria and glomerulosclerosis are avoided.

It will be appreciated that a reduction of proteinuria, in particular in case of an already reduced proteinuria due to an existing medicamentation, should result in a lower frequency of end-stage renal disease and a decrease in mortality rates. The Example illustrates the instant invention and is not meant as limiting the invention to the embodiment specifically described.

### Example:

The study included 23 patients with diabetic nephropathy. The study was double-blind, randomised, and placebo-controlled. All patients were treated with high-dose angiotensin receptor blockers (ARBs) for 4 weeks prior to starting treatment with 5-methyl-pyridine-2-sulfonic acid [6-methoxy-5-(2-methoxy-phenoxy)-2-pyridin-4-yl-pyrimidin-4-yl]-amide (compound A). Patients were included if at the end of the 4-week treatment period with high-dose ARBs, their 24-hour proteinuria was >300 mg/24h. They were randomised into 3 groups: 20 mg of compound A, 50 mg of compound A. or placebo once daily, on top of the high dose ARB. Treatment duration was 4 weeks. The primary variable was 24-hour proteinuria.

Out of the 23 randomised patients, 7 received compound A 20 mg, 8 received compound A 50 mg and 8 received placebo. The mean age (± SD) was similar for all 3 groups. The HbA1c level at entry was also similar for all 3 groups.

### Efficacy

The 24-hour proteinuria data show that for the individual groups, the decrease in proteinuria was -1.0 ± 1.96 g/24 h with compound A 20 mg and -1.3 ± 1.3 g/24 h with compound A 50 mg. The placebo group showed an increase in proteinuria of 0.5 ± 1.78 g/24 h. Although there was a difference in the 24-hour proteinuria at the start of treatment with compound A among the three groups, unlike the placebo group, the 2 groups treated with compound A showed a mean decrease in 24-hour proteinuria of around 1g/24 h, which is clinically relevant.

Compound A used in above Example corresponds to 5-methyl-pyridine-2-sulfonic acid [6-methoxy-5-(2-methoxy-phenoxy)-2-pyridin-4-yl-pyrimidin-4-yl]-amide.

## Claims

1. Use of a compound of formula (I) 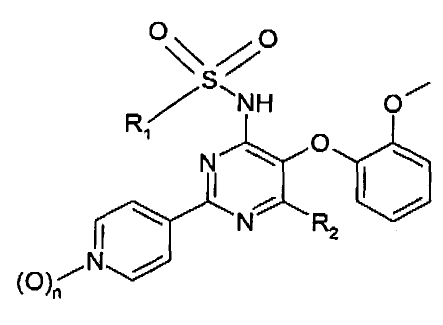 wherein
R₁ is pyridyl or thiazolyl, any of which may optionally be substituted with C₁₋₈alkyl or C₂₋₈alkenyl; and
a) R₂ is methoxy and n is zero or one; or
b) R₂ is chlorine and n is zero
and pharmaceutically acceptable salts thereof
for the manufacture of a medicament for lowering proteinuria.

2. Use of a compound of formula (I) 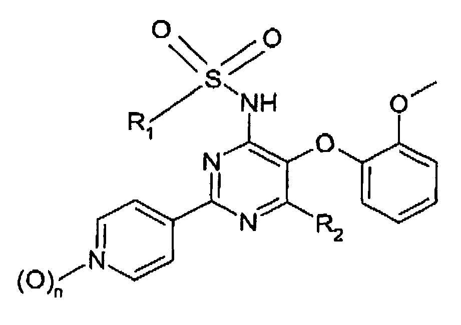 wherein
R₁ is pyridyl or thiazolyl, any of which may optionally be substituted with C₁₋₈alkyl or C₂₋₈alkenyl; and
c) R₂ is methoxy and n is zero or one; or
d) R₂ is chlorine and n is zero
and pharmaceutically acceptable salts thereof
for the manufacture of a medicament for the treatment of diabetic nephropathy.

3. Use according to claim 1 or 2 wherein the compound of formula (I) is 5-methyl-pyridine-2-sulfonic acid [6-methoxy-5-(2-methoxy-phenoxy)-2-pyridin-4-yl-pyrimidin-4-yl]-amide.

4. A pharmaceutical composition comprising
A) a compound of formula (I) 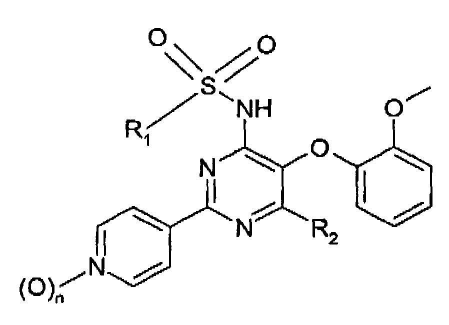 wherein
R₁ is pyridyl or thiazolyl, any of which may optionally be substituted with C₁₋₈alkyl or
C₂₋₈alkenyl; and
a) R₂ is methoxy and n is zero or one; or
b) R₂ is chlorine and n is zero
and pharmaceutically acceptable salts thereof;
B) an angiotensin receptor blocker, an angiotensin-converting enzyme inhibitor, a renin inhibitor, an aldose reductase inhibitor, a proteinkinase C beta- inhibitor, an advanced glycation end product crosslink breaker/inhibitor, sulodexide or an aldosterone receptor antagonist and
C) an excipient.

5. A composition according to claim 4 wherein the compound of formula (I) is 5-methylpyridine-2-sulfonic acid [6-methoxy-5-(2-methoxy-phenoxy)-2-pyridin-4-yl-pyrimidin-4-yl]-amide.

## Patentansprüche

1. Verwendung einer Verbindung der Formel (I) 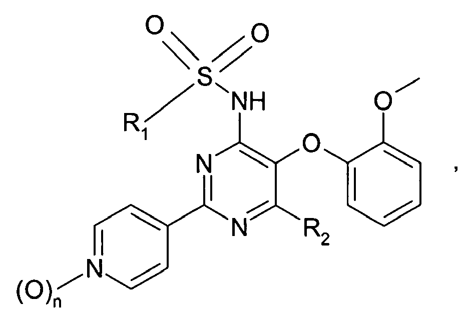 wobei
R₁ Pyridyl oder Thiazolyl ist,
jedes von diesen kann gegebenenfalls mit C₁₋₈Alkyl oder C₂₋₈Alkenyl substituiert sein; und
a) R₂ Methoxy ist und n null oder eins ist; oder
b) R₂ Chlor ist und n null ist,
und deren pharmazeutisch akzetable Salze
zur Herstellung eines Medikamentes zur Verringerung von Proteinurie.

2. Verwendung einer Verbindung der Formel (I) 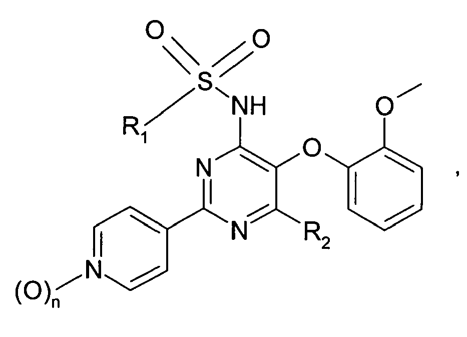 wobei
R₁ Pyridyl oder Thiazolyl ist,
jedes von diesen kann gegebenenfalls mit C₁₋₈Alkyl oder C₂₋₈Alkenyl substituiert sein; und
a) R₂ Methoxy ist und n null oder eins ist; oder
b) R₂ Chlor ist und n null ist
und deren pharmazeutisch akzeptable Salze
zur Herstellung eines Medikamentes zur Behandlung von diabetischer Nephropathie.

3. Verwendung gemäss einem der Ansprüche 1 oder 2, wobei die Verbindung der Formel (I) 5-Methyl-pyridin-2-sulfonsäure-[6-methoxy-5-(2-methoxy-phenoxy)-2-pyridin-4-yl-pyrimidin-4-yl]-amid ist.

4. Eine pharmazeutische Zusammensetzung enthaltend
A) eine Verbindung der Formel (I) 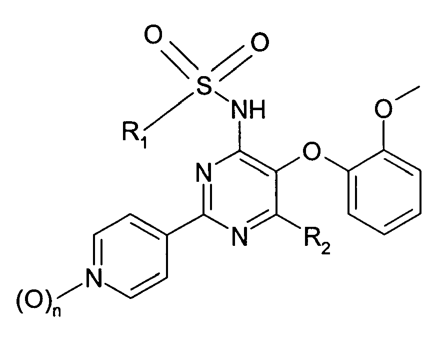 worin
R₁ Pyridyl oder Thiazolyl ist,
jedes von diesen kann gegebenenfalls mit C₁₋₈Alkyl oder C₂₋₈Alkenyl substituiert sein; und
a) R₂ Methoxy ist und n null oder eins ist; oder
b) R₂ Chlor ist und n null ist
und deren pharmazeutisch akzeptable Salze,
B) einen Angiotensin Rezeptor Blocker, einen Angiotensin Umwandlungsenzym Hemmer, einen Reninhemmer, einen Aldose Reduktasehemmer, einen Proteinkinase C beta-Hemmer, einen Quervernetzungbrecher/Inhibitor fortgeschrittener Glykolisierungsendprodukte, Sulodexid oder einen Heparin Rezeptor Antagonist, und
C) einen Excipient.

5. Eine Zusammensetzung gemäss Anspruch 4, wobei die Verbindung der Formel (I) 5-Methyl-pyridin-2-sulfonsäure-[6-methoxy-5-(2-methoxy-phenoxy)-2-pyridin-4-yl-pyrimidin-4-yl]-amid ist.

## Revendications

1. Utilisation d'un composé de formule (I) 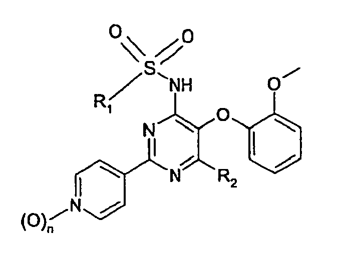 dans laquelle
R₁ est un groupe pyridyle ou un groupe thiazolyle, chacun pouvant être éventuellement substitué par un groupe alkyle en C₁-C₈ ou un groupe alcényle en C₂-C₈ ; et
a) R₂ est un groupe méthoxy et n vaut 0 ou 1 ; ou
b) R₂ est un atome de chlore et n vaut 0 ;
et des sels pharmaceutiquement acceptables de celui-ci,
pour la fabrication d'un médicament pour réduire la protéinurie.

2. Utilisation d'un composé de formule (I) 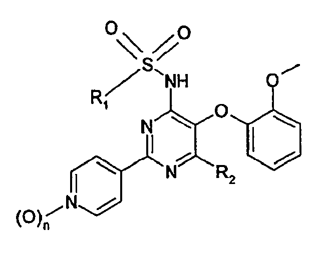 dans laquelle
R₁ est un groupe pyridyle ou un groupe thiazolyle chacun pouvant être éventuellement substitué par un groupe alkyle en C₁-C₈ ou un groupe alcényle en C₂-C₈; et
c) R₂ est un groupe méthoxy et n vaut 0 ou 1 ; ou
d) R₂ est un atome de chlore et n vaut 0 ;
et des sels pharmaceutiquement acceptables de celui-ci,
pour la fabrication d'un médicament pour le traitement d'une néphropathie diabétique.

3. Utilisation selon la revendication 1 ou 2, dans laquelle le composé de formule (I) est le [6-méthoxy-5-(2-méthoxy-phénoxy)-2-pyridin-4-yl-pyrimidin-4-yl]-amide d'acide 5-méthyl-pyridine-2-sulfonique.

4. Composition pharmaceutique comprenant
A) un composé de formule (I) 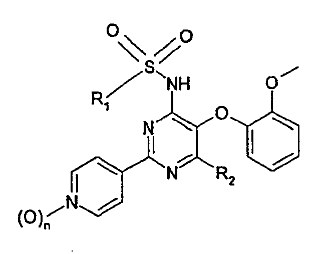 dans laquelle
R₁ est un groupe pyridyle ou un groupe thiazolyle chacun pouvant être éventuellement substitué par un groupe alkyle en C₁-C₈ ou un groupe alcényle en C₂-C₈ ; et
a) R₂ est un groupe méthoxy et n vaut 0 ou 1 ; ou
b) R₂ est un atome de chlore et n vaut 0 ;
et des sels pharmaceutiquement acceptables de celui-ci,
B) un bloqueur des récepteurs de l'angiotensine, un inhibiteur de l'enzyme convertissant l'angiotensine, un inhibiteur de la rénine, un inhibiteur de l'aldose réductase, un inhibiteur de la protéine kinase C β, un inhibiteur/dislocateur de la réticulation du produit final de glycation avancée, le sulodexide ou un antagoniste du récepteur aldostérone, et
C) un excipient.

5. Composition selon la revendication 4, dans laquelle le composé de formule (I) est le [6-méthoxy-5-(2-méthoxy-phénoxy)-2-pyridin-4-yl-pyrimidin-4-yl]-amide d'acide 5-méthyl-pyridine-2-sulfonique.
